(19) Europäisches Patentamt

European Patent Office

Office européen des brevets

(11) **EP 1 649 863 A1**

(12) **EUROPEAN PATENT APPLICATION**
published in accordance with Art. 158(3) EPC

(43) Date of publication:
26.04.2006 Bulletin 2006/17

(21) Application number: 04746681.8

(22) Date of filing: 23.06.2004

(51) Int Cl.:
*A61K 35/74* (1985.01)      *A61P 9/06* (2000.01)
*A61P 43/00* (2000.01)      *A23L 1/28* (1968.09)
*A23C 9/123* (1980.01)      *C12N 1/20* (1980.01)
*C12N 1/16* (1980.01)

(86) International application number:
**PCT/JP2004/009212**

(87) International publication number:
**WO 2004/112809 (29.12.2004 Gazette 2004/53)**

(84) Designated Contracting States:
**AT BE BG CH CY CZ DE DK EE ES FI FR GB GR HU IE IT LI LU MC NL PL PT RO SE SI SK TR**

(30) Priority: **24.06.2003 JP 2003179999**

(71) Applicant: **OTSUKA PHARMACEUTICAL CO., LTD.**
**Chiyoda-ku**
**Tokyo 101-8535 (JP)**

(72) Inventors:
• **KUMEMURA, Megumi**
**Otsu-shi,**
**Shiga, 520-0063 (JP)**
• **IKENAGA, Takeshi**
**520-0031 (JP)**
• **OKAMATSU, Hiroshi**
**830-0061 (JP)**
• **SHIMIZU, Sakayu**
**Kyoto-shi, Kyoto, 616-8212 (JP)**
• **OGAWA, Jun**
**Kita-ku, Kyoto-shi, Kyoto, 603-8051 (JP)**

(74) Representative: **HOFFMANN EITLE**
**Patent- und Rechtsanwälte**
**Arabellastrasse 4**
**D-81925 München (DE)**

(54)  **COMPOSITION FOR LOWERING SERUM URIC ACID LEVEL**

(57)    The present invention provides a composition comprising at least one microorganism selected from the group consisting of lactic acid bacteria and yeasts that have an ability to decompose purines and an action of lowering serum uric acid level and in particular, such a composition in a food, beverage or pharmaceutical form. Such a composition is effective in the prevention and amelioration of hyperuricemia.

EP 1 649 863 A1

**Description**

TECHNICAL FIELD

The present invention relates to novel lactic acid bacterial and yeast strains that have an action of decomposing purines and lowering serum uric acid levels, and to compositions (in the form of foodstuffs, beverages, and pharmaceuticals) containing them.

BACKGROUND OF THE INVENTION

Hyperuricemia is a clinical syndrome manifested by chronic abnormal conditions in which blood uric acid levels are morbidly high and exceed the normal range. Hyperuricemic patients are very likely to develop, in addition to gout, which causes severe pain due to inflammation caused by crystallized uric acid, nephropathy, urinary calculus, cardiovascular disorders, cerebrovascular disorders, etc. Moreover, hyperuricemia is considered to be one of the risk factors for arteriosclerosis.

Hyperuricemia is considered to be caused by the enhancement of uric acid production in the body and by the decreases of urinary excretion of uric acid from the kidney and the gallbladder. Uric acid is a metabolic waste of purine compounds such as adenosine and guanosine, and is generated by metabolism and energy consumption in the body. Therefore, excess intake of purine-rich food leads to increase of serum uric acid levels in human.

The treatment/prevention of hyperuricemia has for a long time involved dietary measures limiting purines contained in food. However, all animal and plant cells contain purines, and most foodstuffs, since animal and plant cells are contained therein, contain purines. Therefore, it is extremely difficult to precisely restrict purine intake, and the restriction of puric acid intake makes it difficult to maintain a nutritional balance provided by food. Moreover, the restriction of purine intake entails the restriction of the intake of inosinic acid, which is one of the purines and known as a tasting component, thereby impairing the taste of food. As a result, it is almost impossible to follow purine-restricting diets for a long period of time.

DISCLOSURE OF THE INVENTION

An object of the present invention is to provide a novel process for preventing/treating hyperuricemia. More specifically, an object of the invention is to provide a novel process for preventing/treating hyperuricemia in which, in contrast to limiting the purine intake from foodstuffs, microorganisms such as lactic acid bacteria and yeasts that decompose purines are orally administered to decompose the purines ingested with food in the intestinal tract, thereby reducing the absorption of the purines and lowering serum uric acid levels, and to provide food and beverage products (fermented milk, lactic acid bacteria beverages, etc.) and pharmaceuticals that can be usefully employed in the process.

The inventors, to achieve the objective described above, investigated the purine decomposing ability of various microorganisms. The inventors then orally administered those microorganisms found to have purine decomposing ability to model animals and measured the serum uric acid levels thereof. As a result, the inventors found the fact that specific lactic acid bacteria and yeasts exhibit the desired uric acid level lowering action. The inventors conducted further research based on this finding and accomplished the present invention.

The present invention provides compositions as recited in Items 1-9, microorganisms as recited in Items 10-13, processes for lowering serum uric acid levels as recited in Items 14-16, and uses of above-mentioned compositions and microorganisms as recited in Items 17-19.

Item 1. A composition comprising at least one microorganism selected from the group consisting of lactic acid bacterial and yeast strains that have an ability to decompose purines and an action of lowering serum uric acid level.

Item 2. A composition according to Item 1, wherein the composition is a serum uric acid level-lowering composition.

Item 3. A composition according to Item 1 or 2, wherein the composition is in the form of a food or beverage.

Item 4. A composition according to Item 3, wherein the composition is a fermented milk, a lactic acid bacteria beverage, a fermented vegetable beverage, a fermented fruit beverage, or a fermented soymilk.

Item 5. A composition according to Item 1 or 2, wherein the composition is a pharmaceutical composition.

Item 6. A composition according to any one of Items 1-5, wherein the microorganism is a lactic acid bacterial strain belonging to the genus *Lactobacillus.*

Item 7. A composition according to Item 6, wherein the lactic acid bacterial strain is one member selected from the group consisting of *Lactobacillus* ONRIC b0185 (FERM BP-10004), *Lactobacillus* ONRIC b0193 (FERM BP-10005), *Lactobacillus* ONRIC b0195 (FERM BP-10006) and *Lactobacillus* ONRIC b0223 (FERM BP-10007).

Item 8. A composition according to any one of Items 1-5, wherein the microorganism is a yeast strain belonging to the genus *Saccharomyces.*

Item 9. A composition according to Item 8, wherein the yeast strain is *Saccharomyces* ONRIC y0046 (FERM BP-10008).

Item 10. A lactic acid bacterial strain belonging to the genus *Lactobacillus* having an ability to decompose purines and an action of lowering serum uric acid level.

Item 11. A lactic acid bacterial strain according to Item 10, wherein the strain is *Lactobacillus* ONRIC b0185 (FERM BP-10004), *Lactobacillus* ONRIC b0193 (FERM BP-10005), *Lactobacillus* ONRIC b0195 (FERM BP-10006) or *Lactobacillus* ONRIC b0223 (FERM BP-10007).

Item 12. A yeast strain belonging to the genus *Saccharomyces* that has an ability to decompose purines and an action of lowering serum uric acid level.

Item 13. A yeast strain according to Item 12, wherein the strain is *Saccharomyces* ONRIC y0046 (FERM BP-10008).

Item 14. A method for lowering a serum uric acid level comprising administering the composition defined in any one of Items 1-9 to a patient in need of a serum uric acid level lowering treatment.

Item 15. A method for lowering a serum uric acid level comprising administering the lactic acid bacterial strain defined in Item 10 or 11 to a patient in need of a serum uric acid level lowering treatment.

Item 16. A method for lowering a serum uric acid level comprising administering the yeast strain defined in Item 12 or 13 to a patient in need of a serum uric acid level lowering treatment.

Item 17. Use of the composition defined in any one of Items 1-9 for lowering a serum uric acid level.

Item 18. Use of the lactic acid bacterial strain defined in Item 10 or 11 for preparing the composition defined in any one of Items 1-9.

Item 19. Use of the yeast strain defined in Item 12 or 13 for preparing the composition defined in any one of Items 1-9.

It is essential that the active ingredient of the serum uric acid level lowering composition of the present invention is a lactic acid bacterial strain or a yeast strain that has an ability to decompose purines and, based on this ability, a serum uric acid lowering action. Such lactic acid bacterial and yeast strains can be isolated and collected from natural products according to the screening methods described below.

The term "lactic acid bacteria" herein refers to microorganisms that produce lactic acid by fermentation. Lactic acid bacteria include strains belonging to the genera *Lactobacillus, Lactococcus, Streptococcus, Enterococcus, Bifidobacterium, Pediococcus* and *Leuconostoc.* Yeasts include strains belonging to the genus *Saccharomyces.*

Preferable examples of lactic acid bacterial and yeast strains that have an action of decomposing purines and lowering serum uric acid levels are strains belonging to the genera *Lactobacillus, Pediococcus* and *Leuconostoc,* and strains belonging to the genus *Saccharomyces.* Among these, particularly preferable are *Lactobacillus* ONRIC b0185 (FERM BP-10004), *Lactobacillus* ONRIC b0193 (FERM BP-10005), *Lactobacillus* ONRIC b0195 (FERM BP-10006), *Lactobacillus* ONRIC b0223 (FERM BP-10007) and *Saccharomyces* ONRIC y0046 (FERM BP-10008), which the inventors newly screened and have deposited.

The term "purine" herein refers to substances that have a purine skeleton. Typical examples include purine nucleotides, purine nucleosides and purine bases. Purine nucleotides include adenylic acid, guanylic acid, inosinic acid, etc. Purine nucleosides include adenosine, guanosine and inosine. Purine bases include adenine, guanine, hypoxanthine and xanthine. Furthermore, purine encompasses oligonucleotides and polynucleotides containing nucleic acids (A and G).

The serum uric acid level lowering composition of the present invention containing the lactic acid bacterial or yeast strain of the invention is described below:

(1) Screening of microorganisms

(1-1) Starting microorganisms

As starting microorganisms, lactic acid bacteria and yeasts isolated from human intestinal contents, vegetable fermentation products and animal food fermentation products are used. Such microorganisms are preserved in the Otsu Nutraceuticals Research Institute of Otsuka Pharmaceutical Co., Ltd.

(1-2) Screening method

Screening for the desired lactic acid bacterial and yeast strains is carried out using as indices (1) an ability to decompose purines (inosine, guanosine, hypoxanthine, xanthine) and (2) an action to lower serum uric acid levels in hyperuricemia model rats. The details of the screening procedures are as described in, for example, Examples 1 and 2 below.

(2) Screened microorganisms

(2-1) *L. fermentum* ONRIC b0185

(a) Macroscopic morphology

(a-1) MRS agar medium

Circular to slightly irregular shape, slight bulging, smooth to slightly rough, white in the center, whitish or achromatic at the periphery.

(a-2) BL agar medium

Irregular, slight bulging, smooth to slightly rough, yellowish brown in the center, whitish brown at the periphery.

(b) Microscopic morphology

Rod-shaped bacterium with no motility. No spore formation.

(c) Growth temperature

Favorable growth at 30-37°C.

(d) Physiological and biochemical characteristics

| | |
|---|---|
| Gram stain | + |
| Glycerol | - |
| Erythritol | - |
| D-Arabinose | - |
| L-Arabinose | + |
| Ribose | + |
| D-Xylose | + |
| L-Xylose | - |
| Adonitol | - |
| β-Methyl-D-Xyloside | - |
| Galactose | + |
| D-Glucose | + |
| D-Fructose | + |
| D-Mannose | ± |
| L-Sorbose | - |
| Rhamnose | - |
| Dulcitol | - |
| Inositol | - |
| Mannitol | ± |
| Sorbitol | - |
| α-Methyl-D-Mannoside | - |
| α-Methyl-D-Glucoside | - |
| N-Acetyl-Glucosamine | - |
| Amygdalin | - |
| Arbutin | - |
| Esculin | - |
| Salicin | - |
| Cellobiose | - |

Table continued

| | |
|---|---|
| Maltose | + |
| Lactose | + |
| Melibiose | + |
| Saccharose | + |
| Trehalose | + |
| Inulin | - |
| Melezitose | - |
| D-Raffinose | + |
| Starch | - |
| Glycogen | - |
| Xylitol | - |
| β-Gentiobiose | - |
| D-Turanose | - |
| D-Lyxose | - |
| D-Tagatose | - |
| D-Fucose | - |
| L-Fucose | - |
| D-Arabitol | - |
| L-Arabitol | - |
| Gluconate | + |
| 2-Keto-Gluconate | - |
| 5-Keto-Gluconate | - |

With reference to the Bergey's Manual of Systematic Bacteriology, this bacterial strain was identified as belonging to *Lactobacillus fermentum* and named *Lactobacillus* ONRIC b0185. The bacterial strain was deposited with the National Institute of Advanced Industrial Science and Technology, International Patent Organism Depositary, AIST Tsukuba Central 6, 1-1 Higashi 1-Chome, Tsukuba-shi, Ibaraki-ken, Japan, on April 15, 2003, under accession no. FERM P-19312, and internationally deposited under accession no. FERM BP-10004 on April 7, 2004.

(2-2) *L. fermentum* ONRIC b0193

(a) Macroscopic morphology

(a-1) MRS agar medium

Circular, conical, smooth to slightly rough, white.

(a-2) BL agar medium

Circular, bulging, smooth to slightly rough, brown.

(b) Microscopic morphology

Rod-shaped bacterium with no motility. No spore formation.

(c) Growth temperature

Favorable growth at 30-37°C.

(d) Physiological and biochemical characteristics

| | |
|---|---|
| Gram stain | + |
| Glycerol | - |
| Erythritol | - |
| D-Arabinose | - |
| L-Arabinose | - |
| Ribose | + |
| D-Xylose | ± |
| L-Xylose | - |
| Adonitol | - |
| β-Methyl-D-Xyloside | - |
| Galactose | + |
| D-Glucose | + |
| D-Fructose | + |
| D-Mannose | + |
| L-Sorbose | - |
| Rhamnose | - |
| Dulcitol | - |
| Inositol | - |
| Mannitol | - |
| Sorbitol | - |
| α-Methyl-D-Mannoside | - |
| α-Methyl-D-Glucoside | - |
| N-Acetyl-Glucosamine | - |
| Amygdalin | - |
| Arbutin | - |
| Esculin | - |
| Salicin | - |
| Cellobiose | - |
| Maltose | + |
| Lactose | + |
| Melibiose | + |
| Saccharose | + |
| Trehalose | - |
| Inulin | - |
| Melezitose | - |
| D-Raffinose | + |
| Starch | - |
| Glycogen | - |
| Xylitol | - |
| β-Gentiobiose | - |
| D-Turanose | - |
| D-Lyxose | - |
| D-Tagatose | - |
| D-Fucose | - |
| L-Fucose | - |
| D-Arabitol | - |
| L-Arabitol | - |
| Gluconate | - |
| 2-Keto-Gluconate | - |
| 5-Keto-Gluconate | - |

With reference to the Bergey's Manual of Systematic Bacteriology, this bacterial strain was identified as belonging to *Lactobacillus fermentum* and named *Lactobacillus* ONRIC b0193. The bacterial strain was deposited with the National Institute of Advanced Industrial Science and Technology, International Patent Organism Depositary, AIST Tsukuba Central 6, 1-1 Higashi 1-Chome, Tsukuba-shi, Ibaraki-ken, Japan, on April 15, 2003, under accession no. FERM P-19313, and internationally deposited under accession no. FERM BP-10005 on April 7, 2004.

(2-3) *L. fermentum* ONRIC b0195

(a) Macroscopic morphology

(a-1) MRS agar medium

Circular, hemispherical, smooth to slightly rough, white.

(a-2) BL agar medium

Circular, bulging, smooth to slightly rough, reddish brown in the center, whitish brown at the periphery.

(b) Microscopic morphology

Rod-shaped bacterium with no motility. No spore formation.

(c) Growth temperature

Favorable growth at 30-37°C.

(d) Physiological and biochemical characteristics

| | |
|---|---|
| Gram stain | + |
| Glycerol | - |
| Erythritol | - |
| D-Arabinose | - |
| L-Arabinose | - |
| Ribose | + |
| D-Xylose | + |
| L-Xylose | - |
| Adonitol | - |
| β-Methyl-D-Xyloside | - |
| Galactose | + |
| D-Glucose | + |
| D-Fructose | + |
| D-Mannose | + |
| L-Sorbose | - |
| Rhamnose | - |
| Dulcitol | - |
| Inositol | - |
| Mannitol | - |
| Sorbitol | - |
| α-Methyl-D-Mannoside | - |
| α-Methyl-D-Glucoside | - |
| N-Acetyl-Glucosamine | - |
| Amygdalin | - |
| Arbutin | - |

Table continued

| | |
|---|---|
| Esculin | - |
| Salicin | - |
| Cellobiose | - |
| Maltose | + |
| Lactose | + |
| Melibiose | + |
| Saccharose | + |
| Trehalose | - |
| Inulin | - |
| Melezitose | - |
| D-Raffinose | + |
| Starch | - |
| Glycogen | - |
| Xylitol | - |
| β-Gentiobiose | - |
| D-Turanose | - |
| D-Lyxose | - |
| D-Tagatose | - |
| D-Fucose | - |
| L-Fucose | - |
| D-Arabitol | - |
| L-Arabitol | - |
| Gluconate | + |
| 2-Keto-Gluconate | - |
| 5-Keto-Gluconate | - |

With reference to the Bergey's Manual of Systematic Bacteriology, this bacterial strain was identified as belonging to *Lactobacillus fermentum* and named *Lactobacillus* ONRIC b0195. The bacterial strain was deposited with the National Institute of Advanced Industrial Science and Technology, International Patent Organism Depositary, AIST Tsukuba Central 6, 1-1 Higashi 1-Chome, Tsukuba-shi, Ibaraki-ken, Japan, on April 15, 2003, under accession no. FERM P-19314, and internationally deposited under accession no. FERM BP-10006 on April 7, 2004.

(2-4) *L. pentosus* ONRIC b0223

(a) Macroscopic morphology

(a-1) MRS agar medium

Circular, hemispherical, smooth, white.

(a-2) BL agar medium

Circular to slightly irregular shape, mesa, smooth to slightly rough, brown in the center, whitish brown at the periphery.

(b) Microscopic morphology

Rod-shaped bacterium with no motility. No spore formation.

(c) Growth temperature

Favorable growth at 30-37°C.

(d) Physiological and biochemical characteristics

| | |
|---|---|
| Gram stain | + |
| Glycerol | + |
| Erythritol | ± |
| D-Arabinose | + |
| L-Arabinose | + |
| Ribose | + |
| D-Xylose | + |
| L-Xylose | + |
| Adonitol | ± |
| β-Methyl-D-Xyloside | ± |
| Galactose | + |
| D-Glucose | + |
| D-Fructose | + |
| D-Mannose | + |
| L-Sorbose | + |
| Rhamnose | + |
| Dulcitol | ± |
| Inositol | ± |
| Mannitol | + |
| Sorbitol | + |
| α-Methyl-D-Mannoside | + |
| α-Methyl-D-Glucoside | + |
| N-Acetyl-Glucosamine | + |
| Amygdalin | + |
| Arbutin | + |
| Esculin | + |
| Salicin | + |
| Cellobiose | + |
| Maltose | + |
| Lactose | + |
| Melibiose | + |
| Saccharose | + |
| Trehalose | + |
| Inulin | ± |
| Melezitose | ± |
| D-Raffinose | + |
| Starch | ± |
| Glycogen | ± |
| Xylitol | ± |
| β-Gentiobiose | + |
| D-Turanose | + |
| D-Lyxose | ± |
| D-Tagatose | ± |
| D-Fucose | - |
| L-Fucose | + |
| D-Arabitol | + |
| L-Arabitol | - |
| Gluconate | + |

Table continued

| | |
|---|---|
| 2-Keto-Gluconate | - |
| 5-Keto-Gluconate | - |

With reference to the Bergey's Manual of Systematic Bacteriology, this bacterial strain was identified as belonging to *Lactobacillus pentosus* and named *Lactobacillus* ONRIC b0223. The bacterial strain was deposited with the National Institute of Advanced Industrial Science and Technology, International Patent Organism Depositary, AIST Tsukuba Central 6, 1-1 Higashi 1-Chome, Tsukuba-shi, Ibaraki-ken, Japan, on April 15, 2003, under accession no. FERM BP-19315, and internationally deposited under accession no. FERM BP-10007 on April 7, 2004.

(2-5) S. *cerevisiae* ONRIC y0046

(a) Macroscopic morphology (YM agar medium)

Circular, bulging, slightly rough to rough, white.

(b) Microscopic morphology

(b-1) Cellular shape

Lemon-like shape

(b-2) Ascospore formation

Present

(b-3) Ascus shape

Spherical

(c) Growth temperature

Favorable growth at 30°C.

(d) Physiological and biochemical characteristics

| | |
|---|---|
| Galactose | + |
| Cyclohexamide | - |
| Saccharose | + |
| N-Acetyl-Glucosamine | - |
| Lactic acid | $\pm$ |
| Arabinose | - |
| Cellobiose | - |
| Raffinose | + |
| Maltose | + |
| Trehalose | - |
| 2-Keto-Calcium Gluconate | - |
| $\alpha$-Methyl-$\alpha$-D-Glucoside | - |
| Mannitol | - |
| Lactose | - |
| Inositol | - |

With reference to the Bergey's Manual of Systematic Bacteriology, this strain was identified as belonging to *Saccharomyces cerevisiae* and named *Saccharomyces* ONRIC y0046. The strain was deposited with the National Institute of Advanced Industrial Science and Technology, International Patent Organism Depositary, AIST Tsukuba Central 6, 1-1 Higashi 1-Chome, Tsukuba-shi, Ibaraki-ken, Japan, on April 15, 2003, under accession no. FERM BP-19316 and internationally deposited under accession no. FERM BP-10008 on April 7, 2004.

(3) Composition of the present invention

It is essential for the composition of the present invention to contain as an active ingredient at least one microorganism selected from the group consisting of lactic acid bacterial and yeast strains that have an action of decomposing purines and lowering serum uric acid levels (hereinafter sometimes referred to as "microorganisms of the invention").

The composition, as with ordinary food compositions and pharmaceutical compositions, is prepared in suitable food and pharmaceutical forms using suitable edible carriers (food materials) and/or pharmaceutically acceptable excipients or diluents.

The microorganisms of the invention to be contained in the composition of the invention are generally viable cells, although they are not limited to such viable cells. For example, culture solutions wherein such microorganisms have been cultured, crude or purified products of cultured microorganisms, freeze-dried products thereof, etc., are usable.

Culture solutions of the aforementioned microorganisms can be prepared, for example, as follows. Specifically, culture solutions can be prepared by introducing microorganisms of the invention and oxygen absorbers into airtight vessels for anaerobic culturing, and culturing for about 48 hours at 28°C using culture media suitable for each microorganism, for example, MRS culture medium for lactic acid bacteria and YM culture medium for yeasts. After the aforementioned culturing, microorganisms can be harvested by, for example, centrifuging the culture solutions at 3000 rotations/minute at 4°C for 10 minutes. Microorganisms can be purified according to known methods. Moreover, the aforementioned culture solutions and cultured products (microorganisms) can be freeze-dried. Such freeze-dried products can be used as the active ingredient of the composition of the present invention.

The composition of the invention may be composed of a culture solution, a cultured product (microorganisms) or those purified or freeze-dried as describe above. The composition may further contain, as necessary, nutrients for the maintenance, proliferation, etc., of the microorganisms of the invention. Specific examples of such nutrients include culture media for culturing the aforementioned microorganisms. Other nutrients include lactosucrose, soy oligosaccharide, lactulose, lactitol, fructooligosaccharide, galactooligosaccharide and like various oligosaccharides. The amount of oligosaccharide to be contained is not limited, and usually suitably selected from a range such that the oligosaccharide is contained in the composition of the invention in a proportion of about 1 to about 3 wt.%.

Various vitamins, trace elements, etc., can be added to the composition of the invention as necessary. Examples of such vitamins include vitamin B, vitamin C, vitamin D, vitamin E, vitamin K and the like. Examples of trace elements include zinc, selenium, etc.

Examples of food and beverage forms of the composition of the invention include fermented milk, lactic acid bacteria beverages, fermented vegetable beverages, fermented fruit beverages, fermented soymilk, etc. The terms "fermented milk" and "lactic acid bacteria beverages" herein are used as defined in Article II(37) "Fermented Milk" and Article II(38) "Lactic Acid Bacteria Beverages" of the "Regulations relating to the Ingredients, etc., of Milks and Milk Products" of the former Japanese Ministry of Health and Welfare. In particular, "fermented milk" refers to a beverage prepared by fermenting milk or a milk product with lactic acid bacteria or yeasts and making it into a paste or solution form. Such fermented milk therefore includes fermented milk in beverage and yogurt forms. The term "lactic acid bacteria beverages" refers to beverages prepared by fermenting milk or milk products with lactic acid bacteria or yeasts, making them into a paste or solution form, and diluting such paste or solution matter (a main ingredient) with water. Fermented vegetable beverages, fermented fruit beverages, and fermented soymilk are described below.

Examples of other food and beverage forms the composition of the invention may take include microorganism-containing microcapsules, solid food items (granules, powders (including fermented milk freeze-dried powders), tablets, effervescent preparation, gums, gummi, puddings, etc.), and milk products other than the aforementioned fermented milk and lactic acid bacteria beverages.

In the composition of the invention in such beverage and food forms, edible carriers can be suitably used to give a taste favorable for the consumption of the composition. For example, preferable carriers are sweeteners and the like that have a taste-improving effect as well as a good mouth feel.

Specific examples of pharmaceutical forms include orally-administrable pharmaceutical forms (aqueous solutions, emulsions, granules, powders, capsules, tablets, etc.) containing pharmaceutically acceptable carriers such as excipients, diluents and the like.

The composition can be prepared in such forms according to known methods. The following items (4) and (5) describe details of the preparation of the composition into such forms as well as details of edible carriers and pharmaceutically acceptable carriers usable during the preparation.

The amount of microorganism contained in the composition of the invention can be suitably selected from a range such that the cell count per 100 g of the composition of the invention is about $10^8$ to about $10^{11}$ (as viable cell count). A cell count can be obtained as follows. A dilute sample is applied to an agar medium for bacterial culture and anaerobically cultured at 37°C, and the colonies thus formed are counted. The amount of microorganism to be contained can be suitably adjusted in view of the aforementioned amount according to the form of the composition of the invention to be prepared, the type of microorganism used, etc.

As described above, since the composition of the invention contain a microorganism (mainly viable cells), use of heating, pressurizing, and like conditions are not preferable in processing of the composition into end products. When the composition of the invention is prepared in a solid food form, it is preferable to use freeze-dried microorganisms directly, or freeze-dried microorganisms treated with a suitable coating agent.

(4) Composition in food or beverage forms

Typical examples of beverage and food forms the serum uric acid reducing composition of the invention can take are fermented milk, lactic acid bacteria beverages, fermented vegetable beverages, fermented fruit beverages, fermented soymilk, etc. Fermented vegetable beverages, fermented fruit beverages and fermented soymilk are described in detail below. Such forms of the composition of the invention can be prepared by culturing microorganisms in suitable fermentation staring materials containing nutrients for the microorganisms, such as fluids derived from vegetables, fruits, or soymilk (emulsified soy), etc., and inducing the fermentation of the staring materials. Vegetables and fruits for use as fermentation starting materials include cuttings, crushings, grindings, squeezed-out juices, enzyme-treated products, and dilutions and concentrates thereof. Vegetables include pumpkins, carrots, tomatoes, bell peppers, celery, spinach, colored sweet potatoes, corn, beats, kale, parsley, cabbages and broccoli. Fruits include apples, peaches, bananas, strawberries, grapes, watermelons, oranges and mandarins.

Cuttings, crushings and grindings of vegetables and fruits can be obtained by, for example, washing the vegetable or fruit, optionally subjecting it to a blanching treatment such as immersion in hot water; and then cutting, pulverizing or milling it by means of a crusher, mixer, food processor, pulverizer, Mycolloider, or the like. Juices can be prepared using a filter press, juicer-mixer, or the like. Juices can also be prepared by filtering the aforementioned grindings through a filter cloth or the like. Enzyme-treated products can be prepared by permitting cellulase, pectinase, protopectinase or the like to act upon the cuttings, crushings, grindings or juices. Dilutions include 1-to 50-fold aqueous dilutions. Concentrates include those concentrated 1- to 100-fold by such means as freeze concentration, concentration under reduced pressure, etc.

Soymilk, which is another specific example of a fermentation starting material, can be prepared from soybean materials according to conventional methods. Soymilk includes, for example, a homogenate prepared by immersing dehulled soybean in water, wet-pulverizing the soybean with a suitable mill such as a colloid mill or the like, and homogenizing the pulverizate in the routine manner, and a solution of water-soluble soy protein in water.

For fermentation using microorganisms, it is preferable to prepare a bulk starter in advance and inoculate a fermentation starting material with the starter. A typical example of bulk starters may be, for example, a culture obtained by inoculating a strain of a microorganism of the invention into a fermentation starting material that has been subjected to usual sterilization at 90 to 121°C for 5 to 20 minutes, yeast extract-supplemented 10% skim milk powder or the like, and incubating the system under the same conditions as described previously. The bulk starter thus prepared usually contains about $10^7$-$10^9$ cells of the microorganism of the invention per gram of the culture.

The fermentation starting material used for the bulk starter may optionally be supplemented with fermentation-promoting substances to give good growth of the microorganism used, for example, glucose, starch, sucrose, lactose, dextrin, sorbitol, fructose and like carbon sources; yeast extract, peptone and like nitrogen sources; vitamins; and minerals.

The inoculum volume of the microorganism should be generally equivalent to a viable cell count of not less than about $1 \times 10^6$, and preferably about $1 \times 10^7$, per cubic centimeter of a fluid containing the fermentation starting material. For culturing conditions, the fermentation temperature is usually selected from the range of about 20 to about 45°C, and preferably about 25 to about 37°C, and the fermentation time is selected from the range of about 5 to about 72 hours.

It should be understood that a lactic acid fermentation product obtained in the above manner may at times have a curd form (a yogurt-like or pudding-like form) and such a product can be directly consumed as a solid food item. A lactic acid fermentation product in such a curd form can be further homogenized to a desired beverage form. Such homogenization can be carried out using an ordinary homogenizer. In particular, it can be carried out using a Gaulin high-pressure homogenizer (LAB 40) at about 200 to about 1000 kgf/cm$^2$, preferably about 300 to about 800 kfg/cm$^2$, or a homogenizer manufactured by Sanwa Machine Industry Corporation (product number: HA x 4571, H20-A2 etc.) at not less than 150 kg/cm$^2$. By such homogenization, beverages with an excellent palatability, particularly a smooth mouth-feel, can be obtained. In carrying out homogenization, it is possible, where necessary, to suitably dilute, add organic acids for pH adjustment, or add various additives which are usually employed in the preparation of beverages, such as sugars, fruit juices, thickeners, surfactants and flavorings in suitable amounts. As a specifically preferable example of each type of additive mentioned above and its amount of addition (% by weight based on the weight of the curd-form fermentation product) are: glucose 8% (% by weight, the same applies hereinafter), sucrose 8%, dextrin 8%, citric acid 0.1%, glycerol fatty acid ester 0.2% and flavoring 0.1%.

The beverages of the invention obtained in a manner described above can be aseptically dispensed into suitable containers in the conventional manner to provide end products. Such products have a good palatability allowing smooth drinking and a good flavor.

The dosage (amount of intake) thereof can be suitably selected according to the age, gender, body weight, status of the disease of the recipient, and other factors, and is not particularly restricted. Generally, it is preferably selected from a range of a microorganism content of $10^6$ to $10^9$ cells/ml. Such a product is generally consumed or administered in an amount of about 50-1000 ml per day.

Another specific example of a food form of the composition of the invention is an effervescent preparation form. The composition in this form can be prepared by formulating 0.01 to 50% (% by weight; the same applies hereinbelow) of microorganism (freeze-dried cells) of the invention and, as effervescent agents, 10 to 35% of sodium carbonate and/or sodium hydrogencarbonate, and 20 to 70% of a neutralizer. A neutralizer usable is an acidic compound capable of neutralizing the aforementioned sodium carbonate and sodium hydrogencarbonate and generating carbon dioxide gas. Typical examples of such compounds are L-tartaric acid, citric acid, fumaric acid, ascorbic acid and like organic acids.

The amount of effervescent agent in the effervescent preparation of the invention is such that when the effervescent preparation of the invention is dissolved in water, the solution shows acidity, particularly an acidity of about pH 3.5 to about pH 4.6. More specifically, the amount can be selected from the range of 10 to 35% sodium carbonate and/or sodium hydrogencarbonate and 20 to 70% neutralizer. In particular, the amount of sodium carbonate is selected from the range of 11 to 31% and preferably 22 to 26%; and sodium hydrogencarbonate from the range of 10 to 35% and preferably 20 and 30%. It is most preferable to use sodium hydrogencarbonate alone within the range of 20 to 25%. The amount of neutralizer is selected from the range of 20 to 70% and preferably 30 to 40%. In particular, it is most preferable to use L-tartaric acid within the range of 20 to 25% and ascorbic acid within the range of 8 to 15%.

The effervescent preparation contains a microorganism of the invention and an effervescent agent as essential components and may optionally contain various known additives such as excipients, binders, disintegrators, lubricants, thickeners, surfactants, osmoregulators, electrolytes, sweeteners, flavorings, colorants, pH regulators, etc. Examples of additives include wheat starch, potato starch, corn starch, dextrin and like starches; sucrose, glucose, fructose, maltose, xylose, lactose and like saccharides; sorbitol, mannitol, maltitol, xylitol and sugar alcohols; coupling sugar, palatinose and like sugar rearrangement oligosaccharides; calcium phosphate, calcium sulfate and like excipients; starches, sugars, gelatin, gum Arabic, dextrin, methylcellulose, polyvinylpyrrolidone, polyvinyl alcohol, hydroxypropylcellulose, gum xanthan, pectin, gum tragacanth, casein, alginic acid and like binders and thickeners; leucine, isoleucine, L-valine, sugar esters, hydrogenated oils, stearic acid, magnesium stearate, talc, macrogols and like lubricants; crystalline cellulose (Avicel manufacture by Asahi Chemical Industry Co., Ltd.), carboxymethylcellulose (CMC), carboxymethylcellulose sodium (CMC-Na), carboxymethylcellulose calcium (CMC-Ca) and like disintegrators; polyoxyethylene sorbitan fatty acid ester (polysorbate), lecithin and like surfactants; aspartame, alitame and like dipeptides; stevia, saccharin and like sweeteners; and the like. Such additives can be suitably selected and used in suitable amounts in consideration of their compatibility with the essential ingredients, properties of the preparation, production method, and other factors.

In addition, vitamins, particularly cyanocobalamine and ascorbic acid (vitamin C), can be contained in the effervescent preparation of the invention in suitable amounts. The amount is not limited but is usually no more than 30% with respect to vitamin C, and it is preferably selected from within the range of about 5 to about 25%.

The method for producing the effervescent preparation of the invention may be basically the same as the usual production methods for effervescent preparations of this kind. In particular, the effervescent tablet form of the preparation of the invention can be prepared by weighing out specific amounts of respective ingredients, mixing them, and processing the whole by the direct powder compression method or the dry or wet granulation-compression method, etc.

The preparation of the invention obtained in such a manner can be converted to a beverage form suitable for oral administration by merely putting it in water and be administered orally.

The dosage (amount of intake) thereof can be suitably selected according to the age, gender, body weight, status of the disease of the recipient, and other factors, and is not particularly restricted. Generally, for each administration, 1 or 2 effervescent tablets of the invention prepared to weigh about 1.5 to about 6.0 g per tablet may be dissolved in 100 to 300 ml of water and administered.

(5) Composition in a pharmaceutical form

The serum uric acid level lowering composition of the invention can be prepared in a general pharmaceutical composition form using a microorganism of the invention as an active ingredient in conjunction with a suitable pharmaceutically acceptable carrier and put into practical use. Examples of such carriers include diluents and excipients such as fillers, extenders, binders, humectants, disintegrators, surfactants, lubricants, etc. These carriers can be suitably selected and used according to the unit dosage form of the preparation to be obtained.

As the unit dosage form of the aforementioned pharmaceutical preparation, a variety of forms can be selected. Typical examples are tablets, pills, powders, solutions, suspensions, emulsions, granules, capsules and suppositories.

In producing tablet forms, examples of pharmaceutical carriers usable include lactose, sucrose, sodium chloride, glucose, urea, starch, calcium carbonate, kaolin, crystalline cellulose, silicic acid, potassium phosphate and like excipients; water, ethanol, propanol, simple syrup, glucose solution, starch solution, gelatin solution, carboxymethylcellulose, hydroxypropylcellulose, methylcellulose, polyvinylpyrrolidone and like binders; carboxymethylcellulose sodium, carboxymethylcellulose calcium, low-substituted hydroxypropylcellulose, dry starch, sodium alginate, agar powder, laminaran powder, sodium hydrogencarbonate, calcium carbonate and like disintegrators; polyoxyethylene-sorbitan fatty acid esters, sodium lauryl sulfate, stearic acid monoglyceride and like surfactants; sucrose, stearin, cacao butter, hydrogenated oils and like disintegration inhibitors; quaternary ammonium bases, sodium lauryl sulfate and like absorption promoters; glycerol, starch and like humectants; starch, lactose, kaolin, bentonite, colloidal silica and like adsorbents; purified talc, stearates, boric acid powder, polyethylene glycol and like lubricants; etc.

Furthermore, tablets can be formulated with conventional coatings if necessary, for example, sugar-coated, gelatin-coated, enteric, or film-coated, double- or multi-layer tablets, etc.

In producing pills, pharmaceutically acceptable carriers include, for example, glucose, lactose, starch, cacao butter, hydrogenated vegetable oil, kaolin, talc, and like excipients; powdered gum arabic, powdered tragacanth, gelatin, ethanol, and like binders; laminaran, agar, and like disintegrants; etc.

Moreover, as necessary, colorants, preservatives, aroma chemicals, flavorings, sweeteners, etc., and other pharmaceuticals can be used in the pharmaceutical compositions of the invention.

The amount of microorganism to be contained in the pharmaceutical preparation of the invention is not limited and can be suitably selected from a broad range. It is usually preferable that the pharmaceutical preparation contains about $10^7$ to about $10^{12}$ cells per unit dosage form.

Administration routes for the aforementioned pharmaceutical preparation are not limited, and can be selected according to the form of the pharmaceutical preparation, age of the patient, gender, severity of the disease, and other conditions. For example, tablets, pills, solutions, suspensions, emulsions, granules, and capsules are administered orally.

Dosage of the pharmaceutical preparation of the invention can be suitably selected according to the application, age of the patient, gender, degree of the disease, and other conditions. Usually, the pharmaceutical preparation is administered such that the active ingredient, i.e., microorganism of the invention, is given in a dose of about 0.5 to about 20 mg/day, per kg body weight. The pharmaceutical preparation may be given in 1 to 4 doses per day.

The composition of the invention is so adapted that, upon ingestion (administration), the microorganism in the composition reaches the lower digestive tract, proliferates and settles as resident microbiota, whereby the expected efficacy is expressed. In this connection, a particularly preferable form of the pharmaceutical preparation is enteric-coated tablets, by which the microorganism can be transported to the intestine without being affected by gastric acid.

The present invention provides a novel composition, particularly a composition in food or pharmaceutical form, effective in the prevention/amelioration of hyperuricemia.

BRIEF DESCRIPTION OF THE DRAWINGS

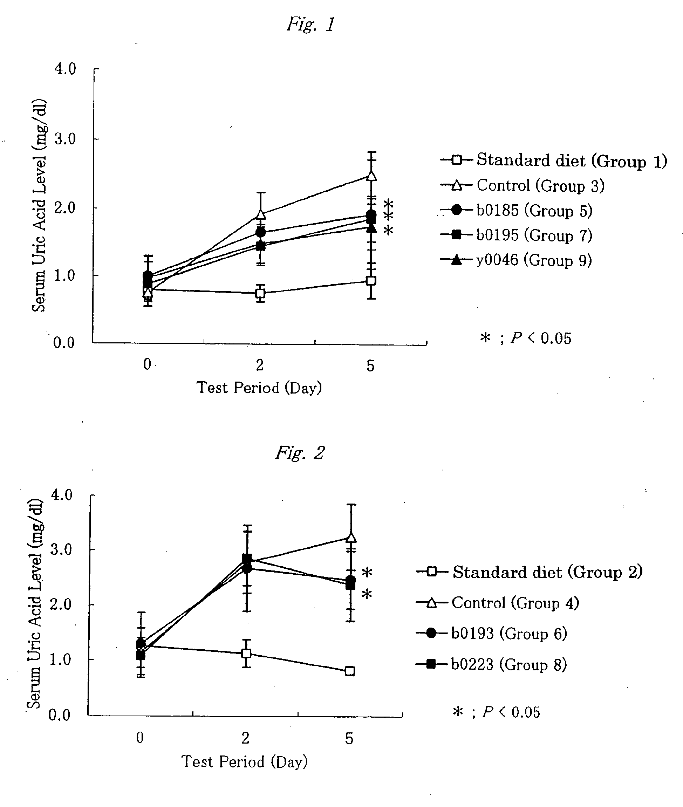

Figures 1 and 2 are graphs showing the changes over time in the serum uric acid levels of test animals used in the test of Example 2 of the present invention.

BEST MODE FOR CARRYING OUT THE INVENTION

Examples are given below to illustrate the present invention in more detail.

Example 1

(1) Test for purine decomposing ability of the microorganisms of the invention

The purine decomposing ability of microorganisms of the invention was evaluated according to the following method.

(1-a) Microorganisms of the invention (ONRIC b0185 (FERM BP-10004), ONRIC b0193 (FERM BP-10005), ONRIC b0195 (FERM BP-10006) and ONRIC b0223 (FERM BP-10007)) and oxygen absorbers (AnaeroPack, manufactured by Mitsubishi Gas Chemical Company, Inc.) for anaerobic culturing were placed in air-tight vessels and anaerobically cultured at 28°C for 48 hours using MRS media for the lactic acid bacterial strains and a YM medium for the yeast strain.

After culturing, microorganisms were harvested by centrifuging the culture solutions at 3000 rotations/minute at 4°C for 10 minutes. Two milliliters of 0.1 M potassium phosphate solution (pH 7.0) containing inosine and guanosine each at a concentration of 1.25 mM was added to each microorganism thus cultured. The microorganism suspensions thus obtained and AnaeroPack were introduced into air-tight vessels and cultured by shaking at 120 rotations/minute at 37°C for 30 minutes.

(1-b) The culture solutions obtained in (1-a) were centrifuged at 3000 rotations/minute at 4°C for 10 minutes. To supernatants (90 μl) were added 10 μl of 0.1 M $HClO_4$ solutions as a reaction terminator. The solutions were then subjected to HPLC. HPLC conditions were as follows:

<Analyzer>

High performance liquid chromatography: LC-6A (manufactured by Shimadzu Corporation)

<Analytical conditions>

| | |
|---|---|
| Column: | Cosmosil 5C$_{18}$-AR (4.6 x 250 mm; Nacalai Tesque, Japan) |
| Carrier buffer: | 100 mM NaClO$_4$ (containing 1% H$_3$PO$_4$) |
| Oven temperature: | 40°C |
| Flow rate: | 1 ml/min |
| Wavelength: | 254 nm |
| Sample amount: | 10 ml |

Inosine, guanosine and metabolites thereof, i.e., xanthine, hypoxanthine, guanine and uric acid, were identified according to the HPLC retention times. These compounds were quantified based on the peak areas of the HPLC chart. Decomposition rates of inosine and guanosine were calculated using the following formula:

```
Decomposition rate = [(1.25 (mM) - A (mM)) / 1.25 (mM)] x 100
```

wherein A is a concentration of inosine or guanosine in the culture supernatants after the reaction.

(1-c) Table 1 shows the results.

Table 1

| Micro-organism | Purine concentration (mM) | | | | | | Rate of purine decomposition (%) | |
|---|---|---|---|---|---|---|---|---|
| | Guanosine | Inosine | Xanthine | Hypoxathine | Guanine | Uric acid | Guanosine | Inosine |
| b0185 strain | - | - | 0.137 | 0525 | - | 0.008 | 100.0 | 100.0 |
| b0193 strain | - | - | 0.059 | 0568 | - | 0.009 | 100.0 | 100.0 |
| b0195 strain | 0.004 | 0.003 | 0.058 | 0.504 | 0.207 | 0.010 | 99.7 | 99.8 |
| b0223 strain | - | 0.022 | - | 0.426 | 0.166 | 0.009 | 100.0 | 98.2 |

It is clear from the results shown in Table 1 that microorganisms of the invention have an ability to decompose purines.

(2) Test for purine decomposing ability of a microorganism of the invention

The purine decomposing ability of a microorganism of the invention was evaluated according to the following method.

(2-a) A microorganism of the invention (ONRIC b0223 (FERM BP-10007)) and an oxygen absorber for anaerobic culturing (AnaeroPack, manufactured by Mitsubishi Gas Chemical Company, Inc.) were placed in an air-tight vessel and anaerobically cultured at 28°C for 48 hours using a MRS medium. After culturing, the microorganism was harvested by centrifuging the culture solution at 3000 rotations/minute at 4°C for 10 minutes. Two milliliters of 0.1 M potassium phosphate solution (pH 7.0) containing hypoxanthine and guanine each at a concentration of 0.25 mM was added to the entire microorganism thus harvested. The microorganism suspension thus obtained and AnaeroPack were introduced into an air-tight vessel and cultured by shaking at 120 rotations/minute at 37°C for 120 minutes.

(2-b) The culture solution obtained in (2-a) was centrifuged at 3000 rotations/minute at 4°C for 10 minutes. To supernatant (90 $\mu$l) was added a 10 $\mu$l of 0.1 M $HClO_4$ solution as a reaction terminator. The solution was then subjected to HPLC. HPLC conditions were as follows:

<Analyzer>

High performance liquid chromatography:    LC-6A (manufactured by Shimadzu Corporation)

<Analytical conditions>

| | |
|---|---|
| Column: | Cosmosil 5C$_{18}$-AR (4.6 x 250 mm; Nacalai Tesque, Japan) |
| Carrier buffer: | 100 mM NaClO$_4$ (containing 1% H$_3$PO$_4$) |
| Oven temperature: | 40°C |
| Flow rate: | 1 ml/min |
| Wavelength: | 254 nm |

Sampled amount:    10 ml

Hypoxanthine, guanine, and metabolites thereof, i.e., xanthine and uric acid were identified according to the HPLC retention times. These compounds were quantified based on the peak areas of the HPLC chart. The decomposition rates of hypoxanthine and guanine were calculated using the following formula:

$$\text{Decomposition rate} = [(0.25 \text{ (mM)} - A \text{ (mM)}) / 0.25 \text{ (mM)}] \times 100$$

wherein A is the concentration of hypoxanthine or guanine in the culture supernatants after the reaction.

(2-c) Table 2 shows the results.

Table 2

| Micro-Organism | Purine concentration (mM) | | | | Rate of purine decomposition (%) | |
|---|---|---|---|---|---|---|
| | Guanine | Hypoxanthine | Xanthine | UricAcid | Guanosine | Inosine |
| b0223 strain | - | - | 0.04 | 0.03 | 100.0 | 100.0 |

It is clear from the results shown in Table 2 that the microorganism of the invention has an ability to decompose purines (ability to decompose hypoxanthine, guanine, and metabolites thereof, i.e., xanthine and uric acid).

Example 2

In this example, food-induced hyperuricemia model animals were prepared according to the method described in *Clinical Toxicology* 13(1), 47-74 (1978), and the effects of microorganisms of the invention upon the serum uric acid levels of model animals were examined according to the method described below.

Microorganisms of the invention ONRIC b0185 (FERM BP-10004), ONRIC b0193 (FERM BP-10005), ONRIC b0195 (FERM BP-10006), ONRIC b0223 (FERM BP-10007) and ONRIC y0046 (FERM BP-10008) were used.

1. Test animals

Six-week-old Wister rats were used (5 rats per group).

2. Feeding conditions

After the arrival of the animals, the animals were naturalized for 1 week. During naturalization, the animals were fed an MF solid diet (manufactured by Oriental Yeast Co., Ltd.), and given tap water freely. Each animal was housed individually in a stainless-steel wire cage. The light-dark cycle consisted of a lighting period from 6:00 to 18:00.

After naturalization, the animals were fed a purified standard diet (referred to as standard diet in Table 3 below) prepared according to AIN-93G (American Institute of Nutrition (1993) AIN-93 purified diets for laboratory rodents: final report of the American Institute of Nutrition *ad hoc* writing committee on the reformulation of the AIN 76A rodent diet, J. *Nutr.* 123: 1939-1951), and given tap water freely for 6 days.

3. Test schedule

After the above 6-day feeding, the animals (7 weeks old) were divided into 8 groups to have the same average body weight among each group and fed the standard diet and the oxonate + RNA diet as shown below for 8 days.

Specifically, (1) the standard diet groups (Groups 1 and 2) were given as a diet AIN-93G (standard diet) as given in the 6-day feeding, and (2) the oxonate + RNA diet groups (Groups 3-9) were given an oxonate/RNA diet prepared to have 2.5 w/w% potassium oxonate and 1.0 w/w% RNA by replacing part of the cornstarch contained in the standard diet with potassium oxonate and RNA. Table 3 below shows the compositions of both diets:

Table 3

|  | Standard diet | Oxonate + RNA diet |
|---|---|---|
| Casein | 200 | 200 |
| L-cystine | 30 | 30 |
| Cornstarch | 397.486 | 362.486 |
| α-Cornstarch | 132 | 132 |
| Sucrose | 100 | 100 |
| Soybean oil | 70 | 70 |
| Cellulose | 50 | 50 |
| AIN-93G mineral mixture | 35 | 35 |
| AIN-93G vitamin mixture | 10 | 10 |
| Tert-butylhydroquinone | 0.014 | 0.014 |
| Potassium oxonate | - | 25 |
| RNA | - | 10 |

The AIN-93G vitamin mixture contained 20 g of choline bitartrate per kg.

Sigma-Aldrich Corporation catalog no. R6625 was used as RNA.

During the test period, the animals of Groups 5-9 were orally forcibly administered every day 1.0 ml of a microorganism suspension prepared by centrifuging a microorganism cultured overnight (b0185, b0193, b0195, b0223 or y0046; microorganism-administered-groups are referred to as b0185 group (Group 5), b0193 group (group 6), b0195 group (Group 7), b0223 group (Group 8) and y0046 group (Group 9)) and mixing the precipitates with physiological saline to have $1.0 \times 10^9$ CFU/ml. The rats from Groups 3 and 4 (control groups) were not administered the aforementioned microorganism suspension.

4. Blood collection

On Day 0, Day 2 and Day 5 of the test feeding, blood was collected from the tail vein of each animal using a syringe. The blood thus obtained was centrifuged at 1500 x g for 20 minutes. The serum was separated and kept at -80°C.

5. Measurement of serum uric acid levels

Serum uric acid levels were measured according to the phosphotungstic acid method using Uric Acid Test Wakos (manufactured by Wako Pure Chemical Industries, Ltd.).

6. Statistical analysis

Results were expressed as mean $\pm$ standard deviation. The significance of serum uric acid levels was determined by the unpaired student's t-test. A $p < 0.05$ was considered significant.

7. Results

Figures 1 and 2 show the serum uric acid levels.

These results clearly show that the rats administered with b0185, b0193, b0195, b0223 or y0046 exhibited serum uric acid levels significantly lower than those of food-induced hyperuricemia model animals.

Example 3

In this example, formulation examples of the composition of the invention are given below.

(1) Preparation of a fermented soymilk

A beverage form of the composition of the invention was prepared by individually weighing out and mixing the ingredients shown in the formulation given below.

| | |
|---|---|
| *Lactobacillus* ONRIC b0185-fermented soymilk | 100 ml |
| Lactosucrose (55% content) | 10.0 g |
| Vitamins and minerals | suitable amount |
| Flavoring | suitable amount |
| Water | suitable amount |
| Total | 150 ml |

The *Lactobacillus* ONRIC b0185-fermented soymilk was prepared by adding $10^8$ cells of *Lactobacillus* ONRIC b0185 (FERM BP-10004) to 1 liter of soymilk (protein content: about 5 g/100 ml) and fermenting at 37°C for 48 hours. Its bacterial cell content was about $1 \times 10^9$ cells/ml.

(2) Preparation of fermented milk

A fermented milk form of the composition of the invention was prepared by individually weighing out and mixing the ingredients shown in the formulation given below.

| | |
|---|---|
| Lactosucrose (55% content) | 10.0 g |
| *Lactobacillus* ONRIC b0193-fermented milk | 100 ml |
| Vitamins and minerals | suitable amount |
| Flavoring | suitable amount |
| Water | suitable amount |
| Total | 150 ml |

The *Lactobacillus* ONRIC b0193-fermented milk was prepared by adding $10^8$ cells of *Lactobacillus* ONRIC b0193 (FERM BP-10005) to 1 liter of cow's milk and fermenting the mixture at 37°C for 24 hours. Its bacterial cell content was about $1 \times 10^8$ cells/ml.

(3) Preparation of fermented milk freeze dried powder

Cow's milk (100 g) was lactic acid-fermented at 37°C for 24 hours using 1 ml of about $10^7$ cells/ml *Lactobacillus* ONRIC b0195 (FERM BP-10006). The fermented product thus obtained (containing the bacterium) was freeze-dried and powdered.

The composition of the present invention in the form of a fermented milk freeze-dried powder was prepared by individually weighing out and mixing the ingredients shown in the formulation given below. Its bacterial cell content was about $1 \times 10^9$ cells/g.

| | |
|---|---|
| Freeze-dried powder of *Lactobacillus* ONRIC b0195-fermented milk | 2.2 g |
| Excipient | suitable amount |
| Vitamins and minerals | suitable amount |
| Flavoring | suitable amount |
| Total | 20 g |

Cornstarch (17 g) was used as the excipient.

(4) Preparation of powder

A powdery form of the composition of the invention was prepared by individually weighing out and mixing the ingredients shown in the formulation given below.

| | |
|---|---|
| Casein | 4.5 g |
| Lactosucrose (55% content) | 10.0 g |
| Freeze-dried powder of *Lactobacillus* ONRIC b0223-fermented milk | 1.0 g |
| Vitamins and minerals | suitable amount |
| Flavoring | suitable amount |
| Total | 20 g |

The freeze-dried powder of *Lactobacillus* ONRIC b0223-fermented milk was prepared by culturing (at 37°C for 24-48 hours) *Lactobacillus* ONRIC b0223 (FERM BP-10007) in 10% aqueous skim milk solution, which serves as a fermentation starting material wherein *Lactobacillus* ONRIC b0223 can proliferate, and freeze-drying the fermented skim milk. Its bacterial cell content was about $10^9$ to $10^{10}$ cells/g.

(5) Preparation of granules

A granular form of the composition of the invention was prepared by individually weighing out and mixing the ingredients shown in the formulation given below.

| | |
|---|---|
| Lactosucrose (55% content) | 10.0 g |
| Freeze-dried powder of *Lactobacillus* ONRIC b0223-fermented milk | 1.0 g |
| Sorbitol | suitable amount |
| Vitamins and minerals | suitable amount |
| Flavoring | suitable amount |
| Total | 20 g |

The freeze-dried powder of *Lactobacillus* ONRIC b0223-fermented milk was the same as used in Example 3-(4)

(6) Microorganism-containing microcapsules

A freeze-dried powder of *Saccharomyces* ONRIC y0046 (FERM BP-10008) having $6 \times 10^{10}$ cells/g prepared by freeze-drying in the same manner as in Example 3-(4) was dispersed in molten hydrogenated palm oil having a melting point of 34°C in conjunction with lactosucrose to give a melt product containing the microorganism, fat/oil, and oligosaccharide mixed in proportions of 25%, 70% and 5%, respectively. This melt product was dropped from the inner-most nozzle of three concentric nozzles at an average flow rate of 0.3 m/s, a liquid mixture of hydrogenated palm oil having a melting point of 43°C and hydrogenated soybean oil was dropped from the center nozzle disposed around the inner-most nozzle at an average flow rate of 0.3 m/s, and a gelatin/pectin solution (85/15 v/v) was dropped from the outer-most nozzle at an average flow rate of 0.3 m/s simultaneously into a cooled and flowing oil, thereby giving three-layer seamless capsules having a diameter of 2.5 mm (1.4 x $10^9$ cells/g capsule).

The weight ratio of capsule content to inner coating to outer coating was 35:35:30.

After air-drying, the capsules were further vacuum dried or vacuum freeze-dried to lower the water activity to an Aw value of 0.20 or lower and the thermal conductivity of 0.16 kcal/mh°C or lower. Aw values were measured by an electric-resistance-type water activity meter (Aw meter, WA-360, Shibaura Electronics Co., Ltd). Thermal conductivities were measured according to the Fitch method.

INDUSTRIAL APPLICABILITY

The present invention provides novel lactic acid bacterial and yeast strains that have a serum uric acid level reducing action due to their ability to decompose purines and a composition in a food, beverage or pharmaceutical form containing them. Such a composition is effective in the prevention and treatment of hyperuricemia.

**Claims**

1. A composition comprising at least one microorganism selected from the group consisting of lactic acid bacterial and yeast strains that have an ability to decompose purines and an action of lowering serum uric acid level.

2. A composition according to Claim 1, wherein the composition is a serum uric acid level-lowering composition.

3. A composition according to Claim 1, wherein the composition is in the form of a food or beverage.

4. A composition according to Claim 3, wherein the composition is a fermented milk, a lactic acid bacteria beverage, a fermented vegetable beverage, a fermented fruit beverage, or a fermented soymilk.

5. A composition according to Claim 1, wherein the composition is a pharmaceutical composition.

6. A composition according to Claim 1, wherein the microorganism is a lactic acid bacterial strain belonging to the genus *Lactobacillus.*

7. A composition according to Claim 6, wherein the lactic acid bacterial strain is one member selected from the group consisting of *Lactobacillus* ONRIC b0185 (FERM BP-10004), *Lactobacillus* ONRIC b0193 (FERM BP-10005), *Lactobacillus* ONRIC b0195 (FERM BP-10006) and *Lactobacillus* ONRIC b0223 (FERM BP-10007).

8. A composition according to Claim 1, wherein the microorganism is a yeast strain belonging to the genus *Saccharomyces.*

9. A composition according to Claim 8, wherein the yeast strain is *Saccharomyces* ONRIC y0046 (FERM BP-10008).

10. A lactic acid bacterial strain belonging to the genus *Lactobacillus* having an ability to decompose purines and an action of lowering serum uric acid level.

11. A lactic acid bacterial strain according to Claim 10, wherein the strain is *Lactobacillus* ONRIC b0185 (FERM BP-10004), *Lactobacillus* ONRIC b0193 (FERM BP-10005), *Lactobacillus* ONRIC b0195 (FERM BP-10006) or *Lactobacillus* ONRIC b0223 (FERM BP-10007).

12. A yeast strain belonging to the genus *Saccharomyces* that has an ability to decompose purines and an action of lowering serum uric acid level.

13. A yeast strain according to Claim 12, wherein the strain is *Saccharomyces* ONRIC y0046 (FERM BP-10008).

14. A method for lowering a serum uric acid level comprising administering the composition defined in Claim 1 to a patient in need of a serum uric acid level lowering treatment.

15. A method for lowering a serum uric acid level comprising administering the lactic acid bacterial strain defined in Claim 10 to a patient in need of a serum uric acid level lowering treatment.

16. A method for lowering a serum uric acid level comprising administering the yeast strain defined in Claim 12 to a patient in need of a serum uric acid level lowering treatment.

17. Use of the composition defined in Claim 1 for lowering serum uric acid level.

18. Use of the lactic acid bacterial strain defined in Claim 10 for preparing the composition defined in Claim 1.

19. Use of the yeast strain recited in Claim 12 or 13 for preparing the composition defined in Claim 1.

*Fig. 1*

—□— Standard diet (Group 1)
—△— Control (Group 3)
—●— b0185 (Group 5)
—■— b0195 (Group 7)
—▲— y0046 (Group 9)

* ; $P < 0.05$

*Fig. 2*

—□— Standard diet (Group 2)
—△— Control (Group 4)
—●— b0193 (Group 6)
—■— b0223 (Group 8)

* ; $P < 0.05$

## INTERNATIONAL SEARCH REPORT

| | International application No. |
|---|---|
| | PCT/JP2004/009212 |

**A. CLASSIFICATION OF SUBJECT MATTER**
Int.Cl⁷ A61K35/74, A61K35/72, A61P9/06, A61P43/00, A23L1/28, A23C9/123, C12N1/20, C12N1/16

According to International Patent Classification (IPC) or to both national classification and IPC

**B. FIELDS SEARCHED**

Minimum documentation searched (classification system followed by classification symbols)
Int.Cl⁷ A61K35/74, A61K35/72, A61P9/06, A61P43/00, A23L1/28, A23C9/123, C12N1/20, C12N1/16

Documentation searched other than minimum documentation to the extent that such documents are included in the fields searched

Electronic data base consulted during the international search (name of data base and, where practicable, search terms used)
CAP(STN), BIOSIS(STN), MEDLINE(STN), EMBASE(STN), WPI, JOIS

**C. DOCUMENTS CONSIDERED TO BE RELEVANT**

| Category* | Citation of document, with indication, where appropriate, of the relevant passages | Relevant to claim No. |
|---|---|---|
| P,X | Takeshi IKENAGA et al., "Takai Purine Nucleoside Taishano o Yusuru Nyusankin no Shokujisei Ko Nyosan Kessho Model Rat no Kecchu Nyosanchi ni Oyobosu Eikyo", Nippon Eiyo Shokuryo Gakkai Sokai Koen Yoshishu", 01 April, 2004 (01.04.04), Vol.58th, page 318, 3I-4p | 1-7,10,11, 18 |
| P,X | Jun OGAWA et al., "Purine Nucleoside Taisha ni Eikyo o Oyobosu Nyusankin no Tansaku", Nippon Nogei Kagakukai 2004 Nendo (Heisei 16 Nendo) Koen Yoshishu, 05 March, 2004 (05.03.04), page 197, 3A15a06 | 1-7,10,11, 18 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 08 September, 2004 (08.09.04) | 19 October, 2004 (19.10.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.

PCT/JP2004/009212

C (Continuation). DOCUMENTS CONSIDERED TO BE RELEVANT

| Category* | Citation of document, with indication, where appropriate, of the relevant passages | Relevant to claim No. |
|---|---|---|
| P,X | Takeshi IKENAGA et al., "Shokujisei Nyosan Kessho Model Rat no Kecchu Nyosanchi ni Oyobosu Nyusankin no Eikyo", Nippon Nogei Kagakukai 2004 Nendo (Heisei 16 Nendo) Koen Yoshishu", 05 March, 2004 (05.03.04), page 197, 3A15a07 | 1-7,10,11,18 |
| A | JP 2003-500451 A (VSL PHARMA LTD.), 07 January, 2003 (07.01.03), Full text; Claims & WO 02/72855 A2          & AU 2000/51016 B &. EP 1181027 A2          & US 2002/61292 A | 1-7,10,11,18 |
| A | CAMPIERI, C. et al., 'Reduction of oxaluria after an oral course of lactic acid bacteria at high concentration.', Kidney Int., (2001), Vol.60, No.3, pages 1097 to 1105 | 1-7,10,11,18 |
| A | WO 02/91833 A1 (KIBOW BIOTECH INC.), 21 November, 2002 (21.11.02), Full text; Claims 15, 16; example 4 & US 2002/187134 A          & EP 1397044 A1 & AU 2002/342641 B | 1-7,10,11,18 |
| A | HOKAMA, S. et al., 'Oxalate-degrading Enterococcus faecalis.' Microbiol.Immunol., (2000), Vol.44, No.4, pages 235 to 240; full text; abstract | 1-7,10,11,18 |
| P,A | DATABASE EMBASE ON STN, (2004) abstract No. 2004081271 & KOZLOWSKI, J. et al., 'The influence of the oral administration of lactic acid bacteria on the oxaluria in the children with calcium-oxalate nephrolithiasis.', Przeglad Pediatryczny, (2003), Vol.33, No.4, pages 313 to 316 | 1-7,10,11,18 |
| P,A | JP 2004-161618 A (Kirin Brewery Co., Ltd.), 10 June, 2004 (10.06.04), Full text (Family: none) | 1-5,8,9,12, 13,19 |
| A | JP 2001-501621 A (PHARMANEX INC.), 06 February, 2001 (06.02.01), Full text; page 14, 3rd line from the bottom & WO 98/14177 A1          & AU 9746587 B & EP 932395 A1 | 1-5,8,9,12, 13,19 |
| A | JP 8-163983 A (Kanebo, Ltd.), 25 June, 1996 (25.06.96), Full text (Family: none) | 1-5,8,9,12, 13,19 |

Form PCT/ISA/210 (continuation of second sheet) (January 2004)

**INTERNATIONAL SEARCH REPORT**

International application No.

PCT/JP2004/009212

C (Continuation).   DOCUMENTS CONSIDERED TO BE RELEVANT

| Category* | Citation of document, with indication, where appropriate, of the relevant passages | Relevant to claim No. |
|---|---|---|
| A | JP 11-318351 A  (Takara Shuzo Kabushiki Kaisha), 24 November, 1999 (24.11.99), Full text (Family: none) | 1-5,8,9,12, 13,19 |
| A | DATABASE CAPLUS ON STN, (1962), No.57:71652 & PORSCHE, T. et al., 'The use of uric acid by Saccharomyces.' Acad.Rep.Populare Romine, Baza Cercetari Stiint Timisoara, Studii Cercetari Stiinte Chim., (1961), Vol.8, Nos.1 to 2, pages 181 to 183 | 1-5,8,9,12, 13,19 |
| A | DATABASE CAPLUS ON STN, (1986), No.104:105897 & MIDDELHOVEN, W.J. et al., 'Yeast species utilizing uric acid, adenine, n-alkylamines or diamines as sole source of carb on and energy.', Antonie van Leeuwenhoek, (1985), Vol.51, No.3, pages 289 to 301 | 1-5,8,9,12, 13,19 |
| A | DATABASE CAPLUS ON STN, (1979), No.90:20791 & ONISHI, H. et al., 'Screening and cultivation of excellent uric acid-utilizable yeasts.', Nippon Nogei Kagaku Kaishi, (1978), Vol.52, No.8, pages 317 to 321 | 1-5,8,9,12, 13,19 |
| A | DATABASE BIOSIS ON STN, (1984), No.1984:261891 & MIDDELHOVE, W.J. et al., 'Growth of Candida-famata and Trichosporon-cutaneum on uric acid as the source of carbon and energy a hitherto unknown property of yeasts.', J.Microbiol., (1983), Vol.49, Nos.4 to 5, pages 361 to 368 | 1-5,8,9,12, 13 |
| A | Jun OGAWA et al., 'Ko Nyosan Kessho Yobo ni Yuko na saikin Purine Nucleosidase no Sho Seishitsu', Nippon Nogei Kagaku Kaishi, (2001), Vol.75, special extra issue, page 366, 3Y6a10 | 1-13,18,19 |

Form PCT/ISA/210 (continuation of second sheet) (January 2004)

## INTERNATIONAL SEARCH REPORT

International application No.

PCT/JP2004/009212

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 14-17
   because they relate to subject matter not required to be searched by this Authority, namely:
   The inventions as set forth in claims 14 to 17 involve embodiments concerning methods for treatment of the human body by therapy and thus relate to a subject matter which this International Searching Authority is not required, under the provisions of Article 17(2)(a)(i) of the PCT   (continued to extra sheet.)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.

PCT/JP2004/009212

Continuation of Box No.II-1 of continuation of first sheet(2)

and Rule 39.1(iv) of the Regulations under the PCT, to search.

\<Subject of search\>

Claims 1 to 5 relate a composition containing as the active ingredient a lactic acid bacterium or a yeast defined by a desired property "capable of degrading purine compounds and lowering serum uric acid level". Although these claims seemingly involve any lactic acid bacteria or yeasts having the above property, only small part of the lactic acid bacteria or yeasts are supported by the description in the meaning within PCT Article 6 and sufficiently disclosed therein in the meaning within PCT Article 5.

Such being the case, the search was made mainly on a lactic acid bacterium belonging to the genus *Lactobacillus* or a yeast belonging to the genes *Saccharomyces* as claimed in any of claims 6 to 9 that belongs to the same genus as the strains practically employed in the description.

Form PCT/ISA/210 (extra sheet) (January 2004)